# EUROPEAN PATENT SPECIFICATION

(11) **EP 1 727 887 B1**
(45) Date of publication and mention of the grant of the patent: **07.05.2008**
(21) Application number: 05717046.6
(22) Date of filing: 15.03.2005
(51) Int. Cl.: C11D 3/395, C11D 3/39, C07D 209/48

(54) **DILUTION PROCESS FOR IMIDOPERCARBOXYLIC ACIDS**
VERDÜNNUNGSVERFAHREN FÜR IMIDOPERCARBONSÄUREN
PROCESSUS DE DILUTION D'ACIDES IMIDOPERCARBOXYLIQUES

(30) Priority: 16.03.2004 IT MI20040497
(43) Date of publication of application: 06.12.2006
(73) Proprietor: Solvay Solexis S.p.A., 20121 Milano (IT)
(72) Inventor: BIANCHI, Ugo, Piero, I-37126 VERONA (IT); GARAFFA, Roberto, I-20141 MILANO (IT)
(74) Representative: Vande Gucht, Anne
(86) International application number: PCT/EP2005/051172
(87) International publication number: WO 2005/090544

(56) References cited:
- EP-A- 0 890 635
- EP-A- 1 010 751
- EP-A- 1 074 607
- WO-A-00/27960
- WO-A-20/04007452

## Description

The present invention relates to a process for diluting concentrated aqueous compositions of imidoalkane-percarboxylic acids, in particular ε-phthalimidoperoxyhexanoic (PAP) acid, in the "β-crystal" form, obtained from imidoalkanepercarboxylic acids in the "α-crystal" form.

The process of the present invention produces dilute compositions that have efficacy levels substantially similar to those of the dilute aqueous compositions known in the prior art having the same concentration of imidoalkanepercarboxylic acids in the β-crystal form, obtained directly from imidoalkanepercarboxylic acids in the α-crystal form.

Solid peroxycarboxylic acids (peracids) that are sparingly water-soluble, among which are imidoalkanepercarboxylic acids, have become increasingly important in detergent compositions since they can be introduced into liquid compositions in their solid form, even in the absence of free hydrogen peroxide. In this way, the advantage of appreciably reducing the chemical interaction of peracids with the other ingredients of the liquid composition is obtained, and any aggressive action with respect to users is limited, to the point that it is contained to within more than tolerable levels. In this way, safe and effective liquid compositions are obtained, which may be used in detergency and disinfecting applications, either by means of spray-metering devices (triggers), which are particularly favoured by consumers. In addition, these water-based liquid compositions meet with increasing commercial interest in detergency and disinfecting applications, since these fields require the use of highly efficient products that allow mild washing conditions to be adopted, for example low temperatures, in particular between 10°C and 20°C, and short washing times, in particular less than 15 minutes: see patent application WO 2004/007452 in the name of the Applicant. The said patent application describes dilute aqueous compositions of imidoalkanepercarboxylic acids in the β-crystal form, obtained directly by starting with imidoalkanepercarboxylic acids in the α-crystal form. These dilute compositions show improved bleaching efficacy, even at working temperatures of the order of from 10°C to 30°C, for industrial and commercial detergency and disinfecting applications. In addition, the imidoalkanepercarboxylic acids contained in these compositions show reduced dissolution times compared with compositions obtained starting with the same peracids in the β-crystal form. The said dissolution times are extremely short, i.e. in less than five minutes more than 99% of the imidoalkanepercarboxylic acids are dissolved in a washing bath at 20°C as described hereinbelow, and fully satisfy the operating requirements as defined by the market for these products.

The term "dissolution time" means the time in which an aliquot of the peracid is dissolved in a standard detergent solution, according to the following method. A weighed sample of the composition is introduced into a solution prepared with water with a hardness of 10°F (French degrees) and containing a standard detergent base, free of bleaching additives (IEC detergent type B, with phosphates - IEC publication 60456) with stirring while thermostatically maintaining a temperature of 20°C; the times at which the samples are taken, measured from the moment of mixing of the two liquid phases, are plotted on a graph on the x-axis, and the areas of the peroxy acid peak, determined by HPLC chromatographic analysis, are plotted on the y-axis; the time corresponding to the dissolution of a given aliquot of peracid is determined from the graph obtained, taking the concentration of peracid obtained asymptotically on the same graph at infinite time (theoretical concentration) as 100% A detailed description of the method, with reference to ε-phthalimidoperoxyhexanoic acid, is given in the examples.

The aqueous peracid compositions that are used for commercial detergency and disinfecting applications preferably have a reduced concentration of peracids, since these active principles are highly efficient.

As mentioned, patent application WO 2004/007452 in the name of the Applicant discloses dilute aqueous compositions of peracids that are used in commercial applications. There are other examples of patent applications which disclose dilute aqueous compositions of peracids that are used in commercial applications. For example, EP 0 890 635 A2 discloses aqueous liquid compositions comprising a diluted amount of percarboxylic acid, especially PAP, as cleaning and hygienizing agent. Other examples are WO 00/27960 and EP 010 75 A2 which disclose aqueous bleaching compositions comprising a diluted amount of peroxycarboxylic acid, especially PAP.

The drawback of these dilute compositions is that, from an economic point of view, in order to limit the costs of transportation of these products from production to sale, it is preferable to transport highly concentrated compositions prepared on an industrial scale and then to dilute them at the point of sale.

The preparation of the concentrated compositions of imidoalkanepercarboxylic acids, in particular ε-phthalimidoperoxyhexanoic acid (PAP), in the β-crystal form, obtained starting with imidoalkanepercarboxylic acids in the α-crystal form, has been described in Italian patent application MI 2004 A 000004 in the name of the Applicant, which is incorporated herein in its entirety by reference. The process for obtaining these concentrated aqueous compositions of imidoalkanepercarboxylic acids comprises the following stages:
■ grinding at a temperature of from 40°C to 65°C crystals of imidoalkanepercarboxylic acids in α form dispersed in an excess of water, preferably at least 2 parts by weight of water/1 part by weight of percarboxylic acid, in the presence of a surfactant chosen from nonionic surfactants;
■ cooling the liquid dispersion to a temperature below 30°C, preferably below 25°C, optionally with the addition of viscosifying additives.

According to the said patent application, in the first step, the grinding is performed in a colloidal mill or in another type of mill provided with a rotor and stator and optionally with radial flow. With this type of process, it is possible to obtain aqueous liquid compositions in the form of dispersions comprising, as percentages by weight, from ≥ 7% to 40% of the said acids and from 0.001% to 0.9% of a surfactant chosen from nonionic surfactants; the difference to 100% consisting of water and of other optional additives for detergent formulations; the concentrated compositions being characterized by a limited viscosity, of less than 2000 mPa.s measured at a temperature of 25°C by applying a shear rate of 20 s⁻¹. The said compositions also show the following combination of properties: a limited viscosity, of less than 2000 mPa.s; high physical and chemical stability in the test of accelerated ageing for seven days at 40°C; optimum bleaching and disinfecting efficacy, attainable with reduced dissolution times for the said imidoalkanepercarboxylic acids.

From the concentrated compositions described in the said patent application, to obtain the dilute compositions required by the market and described in patent application WO 2004/007452, the dilution processes must make it possible to obtain dilute compositions having efficacy levels substantially similar to those of patent WO 2004/007452.

Tests performed by the Applicant have shown that by diluting a concentrated liquid composition of imidoalkanepercarboxylic acids obtained according to Italian patent application MI 2004 A 000004, it is not possible to obtain dilute compositions having efficacy levels substantially similar to the optimum efficacies of the dilute compositions obtained according to patent application WO 2004/007452. In particular, the dissolution time of the dilute compositions that are obtained after diluting the concentrated compositions is still 40% higher than that of the dilute compositions described in patent application WO 2004/007452.

There is a need for a process for diluting concentrated aqueous compositions of imidoalkanepercarboxylic acids in the β-crystal form, obtained from imidoalkanepercarboxylic acids in the α-crystal form, so as to obtain dilute aqueous compositions of the said peracids whose efficacy, in particular as regards the dissolution time, is substantially similar to that of dilute compositions of the same concentration of the same imidoalkanepercarboxylic acids in the β-crystal form obtained directly from the α form.

The Applicant has found, surprisingly and unexpectedly, a process for diluting the above-mentioned concentrated solutions that solves this technical problem.

One subject of the present invention is a process for obtaining dilute aqueous compositions containing an amount of < 7% by weight of imidoalkanepercarboxylic acids, starting with concentrated aqueous compositions containing an amount of ≥ 7% by weight of the said peracids in the β-crystal form, the said concentrated compositions being obtained from the imidoalkanepercarboxylic acids in the α-crystal form, comprising the following steps:
I) dilution of the concentrated aqueous composition of imidoalkanepercarboxylic acids (C) with an aqueous solution (D) having a pH of between 2 and 5, preferably from 3 to 4 and more preferably from 3.2 to 3.7, in a (C):(D) proportion, expressed in parts by weight, of between 0.1:10 and 10:0.2, working at temperatures of between 4°C and 50°C and preferably between 10°C and 25°C;
II) application to the dilute aqueous composition obtained in I) of a shear force of at least 5000 s⁻¹, until a constant dynamic viscosity is obtained;
III) dilution of the aqueous composition obtained in II) with water to obtain an imidoalkanepercarboxylic acid concentration of < 7% by weight;
IV) optionally, final homogenization of the composition, for example by stirring the composition.

The imidoalkanepercarboxylic acids that are contained in the dilute compositions of the present invention have the formula (I) 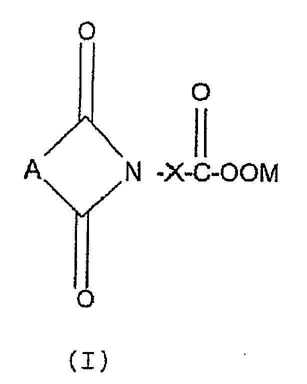 in which A indicates a group chosen from the following: 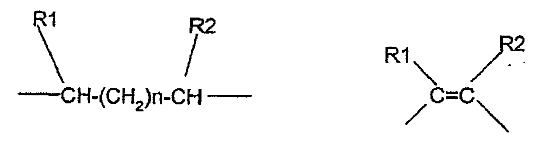 or 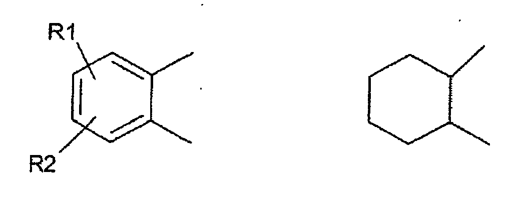 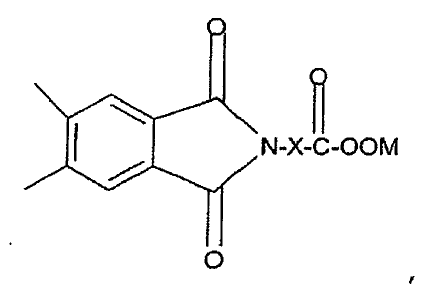 in which:
n is an integer 0, 1 or 2,
R1 has one of the following meanings: hydrogen, chlorine, bromine, C₁-C₂₀ alkyl, C₂-C₂₀ alkenyl, aryl or alkylaryl,
R2 is hydrogen, chlorine, bromine or a group chosen from the following: -S0₃M, -CO₂M,-CO₃M or -OSO₃M,
M means hydrogen, an alkali metal, ammonium or an equivalent of an alkaline-earth metal,
X indicates a C₁-C₁₉ alkylene or an arylene.
ε -Phthalimidoperoxyhexanoic acid is preferably used as imidoalkanepercarboxylic acid.

The imidoalkanepercarboxylic acids are in the β-crystal form.

The imidoalkanepercarboxylic acids contained in the dilute compositions obtained by the process of the invention have a dissolution time t₉₉ in the washing bath, as defined below, at a temperature of 20°C, of less than five minutes.

The dissolution time t₉₉ is determined by dispersing a sample of the dilute liquid composition in a solution prepared with water with a hardness of 10°F (French degrees) and containing a standard detergent base, free of bleaching additives (IEC detergent type B, with phosphates - IEC publication 60456), with stirring while thermostatically maintaining a temperature of 20°C; the times at which the samples are taken, measured from the moment of mixing of the two compositions, are plotted on a graph on the x-axis, and the areas of the peracid peak, determined by HPLC chromatographic analysis, are plotted on the y-axis; the time t₉₉ at which the amount of dissolved peracid corresponds to 99% of the peracid present, which is determined from the peracid concentration obtained asymptotically on the same graph at infinite time, being taken from the graph thus obtained. A detailed description of the method is given in the examples.

The crystalline form of the imidoalkanepercarboxylic acids referred to herein as the β form is the form known in the prior art for the said peracids, since it may be obtained directly by crystallization of the said compounds.

The imidoalkanepercarboxylic acids may be obtained via methods known in the prior art: see, for example, European patents EP 325 289, EP 325 288, EP 490 409, EP 560 155, EP 556 769 and EP 780 374.

As regards the concentrated aqueous compositions of peracids used in step I), concentrated compositions of imidoalkanepercarboxylic acids in β form obtained from the corresponding α form of these peracids, as described in patent application MI 2004 A 000004 in the name of the Applicant, are preferably used in the present invention: see that mentioned above.

In step I), the aqueous solution (D) that is used may be a buffer solution. Optionally, a chelating and/or sequestering agent may be added, in an amount of from 0.005% to 5% by weight relative to the total weight of the composition. Chelating and/or sequestering agents that may be mentioned include quinoline and salts thereof alkali metal polyphosphates, picolinic acid and dipicolinic acid, monophosphonic or polyphosphonic acids, for example and preferably 1-hydroxyethylidene-1,1-diphosphonic acid (HEDP).

Nonionic and/or anionic surfactants may also optionally be added in step I); the total concentration of the said surfactants, expressed as a percentage by weight, is not less than 0.001 %.

As examples of nonionic surfactants, see the book "Nonionic surfactants", Ed. M.J. Schick, Marcel Dekker 1967, pp 76-85 and 103-141. Examples that may be mentioned include ethoxylated, polyethoxylated, propoxylated or polypropoxylated nonionic surfactants or surfactants containing one or more propoxy repeating units and one or more ethoxy units. Examples of these surfactants are the surfactants known commercially under the trade names Triton® X100 (Dow), Tergitol® TMN100x (Dow), Antarox® 863 (Rhodia), Rhodasurf® 870 (Rhodia), Genapol® X080 (Clariant), Genapol® X020 (Clariant), Genapol® X060 (Clariant), Genapol® X040 (Clariant) and Lutensol® XL40 (BASF).

The anionic surfactants that are preferably used are sulfonates of at least C₈ saturated alkyl chains, in particular secondary alkyl sulfonates with the sulfonic group in a chain, for instance Hostapur® SAS from Clariant.

In step II) of the process of the invention, the time for which the shear force is applied generally ranges from about five minutes to about two hours. Generally, step II) is performed at a temperature not above 50°C, preferably not above 35°C and even more preferably not above 25°C, until a constant dynamic viscosity is obtained, which is measured at the temperature at which the treatment is performed.

Step II) may be performed, for example, in a colloidal mill, optionally with recycling of the treated formulation. The process may also be performed at very high shear, of about 50 000 s⁻¹ or more, and even of the order of from 70 000 up to 100 000 s⁻¹, in the case where it is desired to use short treatment times.

It has been found by the Applicant that step II) may also be performed at lower shear, for example of the order of 5000-30 000 s⁻¹, maintaining short treatment times, if the concentration of the nonionic and anionic surfactants present, expressed as a percentage by weight, is not less than 0.01% and preferably not less than 0.03%. Nonionic and/or anionic surfactants may optionally be added in this phase.

In step III), the dilute compositions of imidoalkanepercarboxylic acids that are obtained preferably have a concentration of not less than 0.1 % by weight.

In step III), the dilution may optionally be performed with an aqueous solution of a suspending agent having a concentration of between 0.1 % and 5% by weight and preferably from 0.5% to 3% by weight, until a concentration of suspending agent, expressed as a percentage by weight relative to the total weight of the composition, of at least 0.01% is obtained

The suspending agent is added so as to have a final concentration in the dilute composition generally of from 0.01 % to 0.6% and preferably from 0.05% to 0.3%. Xanthan gum is preferably used.

The suspending agent is preferably used in step III) in the process of the invention, since it has been found that the physical stability of the dilute composition is improved..

Step III) is generally performed at a temperature of from 5°C to 35°C and preferably at a temperature of about 20°C.

As mentioned, the dilute compositions obtained by the process of the present invention have efficacy levels that are substantially similar to those of the dilute aqueous compositions of imidoalkanepercarboxylic acids known in the prior art, having the same content of peracid in the β-crystal form, obtained directly from the peracids in the α-crystal form.

As already mentioned, the liquid compositions based on imidoalkanepercarboxylic acids, obtained by the process of the present invention, are used for bleaching and disinfecting applications intrinsic to the detergency market, both for industrial use and for domestic use. The compositions of the present invention are particularly suitable for bleaching, in particular for removing marks from any type of fabric, either white or coloured, leaving unimpaired the characteristics of the fabric that has been subjected to the treatment. In addition, the compositions of the invention constitute an effective vehicle for imidoalkanepercarboxylic acids and thus a convenient form of disinfectant preparations, which are particularly favoured in the detergency market, precisely for the gradual prevalence of mild washing conditions, the said washing always being performed at low temperature and over short washing times, which, per se, would favour the proliferation of the bacterial load and be harmful to the hygiene. The said disinfectant solutions also find a useful application in the field of the cleaning and sanitization of hard surfaces. The dilute liquid compositions based on imidoalkanepercarboxylic acids obtained by the process of the present invention may be applied by spray-metering means (triggers), which are particularly favoured by consumers.

The examples that follow are given as non-limiting illustrations of the present invention.

### Examples

### Determination of the PAP titre

The analysis is performed by iodometric titration, by titration with sodium thiosulfate of the iodine that is released from the reaction of the potassium iodide with the peracid in the compositions, according to the following method. An accurately weighed amount of 500 mg of the composition is diluted in 100 ml of water, and 10 ml of glacial acetic acid and 30 ml of aqueous 10% w/w potassium iodide solution are then added. The iodine produced from the reaction is titrated with an aqueous sodium thiosulfate solution of known titre, using a Mettler® DL 40 potentiometric titrator equipped with a platinum electrode and a reference electrode.

### Determination of the rate of dissolution of the PAP of a formulation in an aqueous solution of a standard detergent base

The rate of dissolution is determined by the following method.

A sample of 500 mg of the composition is dispersed in one litre of solution prepared with water with a hardness of 10°F and 1.70 g of standard detergent base, free of bleaching additives (IEC detergent type B, with phosphates - IEC publication 60456), kept stirred and thermostatically regulated at a temperature of 20°C. Successive samples of liquid phase, carefully filtered through a 0.45 micron filter, are taken. The times at which the samples are taken, measured from the moment of mixing of the two compositions, are plotted on a graph on the x-axis. The times used, expressed in minutes, were as follows: 1, 3, 5, 10, 15, 30, 60, 120. The areas of the PAP peak, determined by HPLC analysis, are plotted on the y-axis of the graph. The times at which the amount of dissolved PAP corresponds, respectively, to 80% (t₈₀), 90% (t₉₀) and 99 (t₉₉) of the peroxy acid present, determined by taking the concentration of PAP obtained asymptotically at infinite time (theoretical concentration) as 100%, are determined from the graph obtained.

### Bleaching test

The test is performed by adding 500 mg of the composition to one litre of 2% sodium carbonate solution coloured with 0.035% of eriochrome black, leaving the mixture to stand (without stirring) at 20°C for five minutes. The test is positive if the dispersion discolours.

### Determination of the dynamic viscosity

The viscosity was determined using a TA Instruments® model AR 500 rotary viscometer, at a temperature of 25°C, at a shear rate of 20 s⁻¹, using a parallel spindle 4 cm in diameter.

### Example 1 Comparative

### Production of a water-based dilute liquid composition with a PAP content of 5% by weight, having a high rate of dissolution

12.20 kg of technical-grade PAP of α form, prepared according to patent application WO 2004/007452, Example 5B, 0.20 kg of xanthan gum, 0.66 kg of Hostapur® SAS anionic surfactant and 0.10 kg of antifoam DB100 are dispersed in 188.70 kg of demineralized water at 50°C and treated for five minutes with a Silverson machine, and then by mechanical paddle stirring for 30 minutes at 50°C, and then for 30 minutes at room temperature. The composition obtained is an aqueous slurry with an active PAP titre of 5.0%.

The following parameters were determined on the composition obtained: the viscosity, bleaching test, dissolution times T₈₀, T₉₀, T₉₉.

The results of the determinations performed on the aqueous composition are given in Table 1.

### Example 2 Comparative

### Production of a water-based concentrated liquid composition with a PAP content of 20% by weight, having a high rate of dissolution

The following components:
■ HEDP (1-hydroxyethylidene-1,1-diphosphonic acid) Sequion 10H60 (aqueous 60% solution - Bozzetto) 66.8 g (final concentration: 1.00% by weight);
■ sodium hydroxide (solution at 50% by weight) 14.4 g;
■ Genapol X020 polyethoxylated (2-5 EO) nonionic surfactant (Clariant) 2,0 g (final concentration: 0.05% by weight)
■ are added in order into a 10 litre jacketed container containing 2368.0 g of water, the liquid phase being kept stirred by means of a variable-speed motor set at 120 revolutions per minute and equipped with an anchor-stirring shaft.

The solution obtained is heated and maintained at a constant temperature of 45°C by means of a water-circulating thermostat connected to the jacket.

With stirring, PAP in α-crystal form, prepared according to the patent application in the name of the Applicant WO 2004/007452, is fed in in a total amount of 956.0 g (final concentration of the composition: 20% by weight) over a period of at least 60 minutes, by means of small successive additions. During the addition, the mass is kept stirred at a temperature of 45°C, and is simultaneously sent for grinding in a colloidal mill or in a Silverson mill. Five minutes after the end of the addition of PAP the grinding is stopped and stirring is continued for a further 60 minutes.

The temperature of the thermostatic bath is lowered to 20°C and the mass is left for the time required for it to cool down. 600.0 g of a solution containing 2% by weight of xanthan gum (final concentration: 0.3% by weight) are then added. The product is left to homogenize by gentle stirring for ten minutes.

The results of the determinations performed on the aqueous composition are given in Table 1.

### Example 2A Comparative

### Dilution to 5% by weight of PAP of the 20% concentrated aqueous composition of Comparative Example 2

1 kg of water-based liquid composition with a PAP content of 20%, prepared in the preceding example, is transferred into a 5 litre beaker and 50.00 g of HEDP; 10.80 g of sodium hydroxide (solution at 50% by weight); 50.0 g of a solution containing 2% by weight of xanthan gum (final concentration: 0.1%) and 2890.0 g of water are added, with stirring (shear of about 500 s⁻¹).

4 kg of a dilute aqueous composition containing 5% PAP are obtained.

The results of the determinations performed on the aqueous formulation are given in Table 1.

### Example 3

### Dilution to 5% by weight of PAP of the 20% concentrated aqueous composition of Comparative Example 2 using the process of the present invention with high shear (without addition of surfactants)

1 kg of water-based concentrated liquid composition with a PAP content of 20%, prepared in Comparative Example 2, is transferred into a 5 litre beaker and 50.00 g of HEDP; 10.80 g of sodium hydroxide (solution at 50% by weight) and 1890 g of water are added into a colloidal mill operating at a shear of 50 000 s⁻¹. The temperature during the treatment is maintained at between 20°C and 25°C. Recycling is performed in the mill for 30 minutes, and the treatment is then complete. Next, after the dispersion has been transferred into a beaker, 250.0 g of a solution containing 2% by weight of xanthan gum (final concentration: 0.2%) and 800.0 g of water are added with stirring. The dilution is performed at room temperature.

4 kg of a dilute aqueous composition containing 5% PAP are obtained.

The results of the determinations performed on the aqueous formulation are given in Table 1.

### Example 4

### Dilution to 5% by weight of PAP of the 20% concentrated aqueous composition of Comparative Example 2, using the process of the present invention, with addition of a nonionic surfactant, using a lower shear than that in Example 3

1 kg of water-based concentrated liquid composition with a PAP content of 20%, prepared in Comparative Example 2, is transferred into a 5 litre beaker and 50.00 g of HEDP; 10.80 g of sodium hydroxide (solution at 50% by weight); 2.0 g of Genapol X020 polyethoxylated (2-5 EO) nonionic surfactant (Clariant) (final concentration of the nonionic surfactant: 0.06%) and 1888 g of water are added, in a colloidal mill operating at a shear of 20 000 s⁻¹. The temperature during the treatment is maintained at between 20°C and 25°C. Recycling is performed in the mill for 30 minutes, and the treatment is then complete. Next, after the dispersion has been transferred into a beaker, 250.0 g of a solution containing 2% by weight of xanthan gum (final concentration: 0.2%) and 800.0 g of water are added with stirring. This step is performed at room temperature (20°C).

4 kg of a dilute aqueous composition containing 5% PAP are obtained.

The results of the determinations performed on the aqueous composition of Example 4 are given in Table 1.

### Example 5

### Dilution to 5% by weight of PAP of the 20% concentrated aqueous PAP composition of Comparative Example 2,using the process of the present invention, with addition of an anionic surfactant, using a lower shear than that in Example 3

1 kg of water-based concentrated liquid composition with a PAP content of 20%, prepared in Comparative Example 2, is transferred into a 5 litre beaker and 50.00 g of HEDP; 10.80 g of sodium hydroxide (solution at 50% by weight); 6.67 g of Hostapur® SAS anionic surfactant (secondary alkanesulfonate) (Clariant) (final concentration of the anionic surfactant: 0.06%) and 1888 g of water are added, in a colloidal mill operating at a shear of 20 000 s⁻¹. The temperature during the treatment is maintained at between 20°C and 25°C. Recycling is performed in the mill for 30 minutes, and the treatment is then complete. Next, after the dispersion has been transferred into a beaker, 250.0 g of a solution containing 2% by weight of xanthan gum (final concentration: 0.2%) and 800.0 g of water are added with stirring. This step is performed at room temperature (20°C).

4 kg of a dilute aqueous composition containing 5% PAP are obtained.

The results of the determinations performed on the aqueous composition of Example 5 are given in Table 1.

**TABLE 1**

| Determination of the viscosity, bleaching test and dissolution times t₈₀, t₉₀ and t₉₉, at a temperature of 20°C, of the aqueous | | | | | | | |
|---|---|---|---|---|---|---|---|
| formulations of Comparative Examples 1, 2 and 2A and of Examples 3-5 | | | | | | | |
| Ex. | PAP conc. | Shear | Viscosity | Bleaching | Dissolution times | | |
| | Weight % | s⁻¹ | mPa.s | test | at 20°C | | |
| | | | | | min | | |
| | | | | | t₈₀ | t₉₀ | t₉₉ |
| 1 Comparative | 5 | - | 300 | positive | < 1 | 1 | 3 |
| 2 Comparative | 20 | - | 734 | positive | 2 | 2.9 | 7.5 |
| 2A Comparative | 5 | - | 350 | positive | 2 | 2.5 | 7.5 |
| 3 | 5 | 50000 | 150 | positive | < 1 | 1 | 3.5 |
| 4 | 5 | 20000 | 120 | positive | < 1 | 1 | 3.6 |
| 5 | 5 | 20 000 | 270 | positive | < 1 | < 1 | 3.2 |

## Claims

1. Process for obtaining dilute aqueous compositions containing an amount of < 7% by weight of imidoalkanepercaxboxylic acids, starting with concentrated aqueous compositions containing an amount of ≥ 7% of the said peracids in the β-crystal form, the said concentrated compositions being obtained from the imidoalkanepercarboxylic acids in the α-crystal form, comprising the following steps:
I) dilution of the concentrated aqueous composition of imidoalkanepercarboxylic acids (C) with an aqueous solution (D) having a pH of between 2 and 5, preferably from 3 to 4 and more preferably from 3.2 to 3.7, in a (C):(D) proportion, expressed in parts by weight, of between 0.1:10 and 10:0.2, working at temperatures of between 4°C and 50°C and preferably between 10°C and 25°C;
II) application to the dilute aqueous composition obtained in I) of a shear force of at least 5000 s⁻¹, until a constant dynamic viscosity is obtained;
III) dilution of the dilute aqueous composition obtained in II) to obtain an imidoalkanepercarboxylic acid concentration of < 7% by weight;
IV) optionally, final homogenization of the composition.

2. Process according to Claim 1, in which, the imidoalkanepercarboxylic acids have the formula (I) 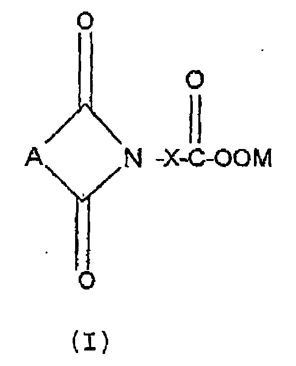 in which A indicates a group chosen from the following: 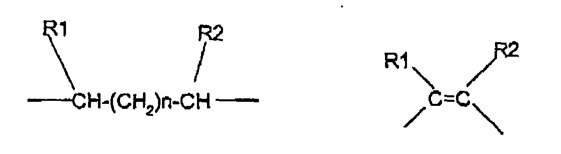 or 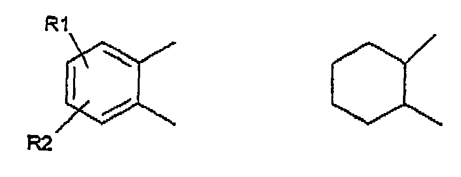 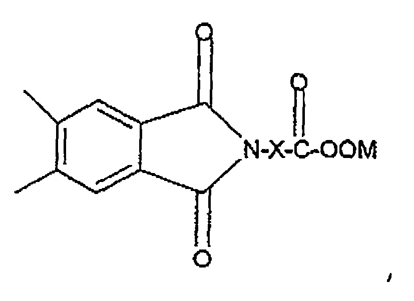 in which:
n is an integer 0, 1 or 2,
R1 has one of the following meanings: hydrogen, chlorine, bromine, C₁-C₂₀ alkyl, C₂-C₂₀ alkenyl, aryl or alkylaryl,
R2 is hydrogen, chlorine, bromine or a group chosen from the following: -SO₃M, -CO₂M, -CO₃M or -OSO₃M,
M means hydrogen, an alkali metal, ammonium or an equivalent of an alkaline-earth metal,
X indicates a C₁-C₁₉ alkylene or an arylene.

3. Process according to Claim 2, in which the imidoalkanepercarboxylic acid is ε-pblthalixnidoperoxyhexanoic acid.

4. Process according to Claims 1 to 3, in which the imidoalkanepercarboxylic acids contained in the dilute compositions have a dissolution time t₉₉ in the washing bath at a temperature of 20°C of less than five minutes.

5. Process according to Claims 1 to 4, in which, in step I), nonionic and/or anionic surfactants are added such that the total concentration of surfactants, expressed as a percentage by weight, is not less than 0.001%.

6. Process according to Claims 1 to 5, in which, in step II), a shear of between 50 000 and 100 000 s⁻¹ is used.

7. Process according to Claims 1 to 5, in which, in step II), a shear of 5000-30 000 s⁻¹ is used in the presence of a total amount of nonionic and/or anionic surfactants, expressed as a percentage by weight, of not less than 0.01% and preferably not less than 0.03%.

8. Process according to Claims 1 to 7, in which, in step III), the dilution is performed with an aqueous solution of a suspending agent so as to have a final concentration in the dilute composition of from 0.01 % to 0.6% and preferably from 0.05% to 0.3% of suspending agent.

9. Process according to Claim 8, in which the suspending agent is xanthan gum.

10. Process according to Claims 8 and 9, in which step III) is performed at a temperature of between 5°C and 35°C.

11. Dilute aqueous compositions containing an amount of < 7%, expressed as a percentage by weight, of imidoalkanepercarboxylic acids, obtainable by the process of anyone of claims 1 to 10.

12. Use of the dilute aqueous compositions according to Claims 1 in detergency applications.

13. Use of the dilute aqueous compositions according to Claims 11 in disinfection applications.

14. Use of the dilute aqueous compositions according to Claims 12 or 13, in which the dilute aqueous compositions are applied by means of disperses (triggers).

15. Use of the dilute aqueous compositions according to Claims 12 to 14, used in detergency applications at a temperature of from about 10°C to 30°C.

## Patentansprüche

1. Verfahren zum Erhalt verdünnter wäßriger Lösungen, die eine Menge von < 7 Gew.-% Imidoalkanpercarbonsäuren enthalten, ausgehend von konzentrierten wäßrigen Zusammensetzungen, die eine Menge von≥7 Gew.-% der Persäuren in derβ-Kristallform enthalten, wobei die konzentrierten Zusammensetzungen aus den Imidoalkanpercarbonsäuren in derα-Kristallform erhalten werden, bei dem man:
I) die konzentrierte wäßrige Zusammensetzung von Imidoalkanpercarbonsäuren (C) mit einer wäßrigen Lösung (D) mit einem pH-Wert zwischen 2 und 5, vorzugsweise 3 bis 4 und besonders bevorzugt 3,2 bis 3,7, in einem (C):(D)-Verhältnis, ausgedrückt in Gewichtsteilen, zwischen 0,1:10 und 10:0,2 verdünnt, wobei man bei Temperaturen zwischen 4°C und 50°C und vorzugsweise zwischen 10°C und 25°C arbeitet;
II) die in I) erhaltene verdünnte wäßrige Zusammensetzung einer Scherkraft von mindestens 5000 s⁻¹ unterwirft, bis sich eine konstante dynamische Viskosität ergibt;
III) die in II) erhaltene verdünnte wäßrige Zusammensetzung auf eine Imidoalkanpercarbonsäure-Konzentration von < 7 Gew.-% verdünnt;
IV) gegebenenfalls die Zusammensetzung abschließend homogenisiert.

2. Verfahren nach Anspruch 1, bei dem die Imidoalkanpercarbonsäuren die Formel (I) 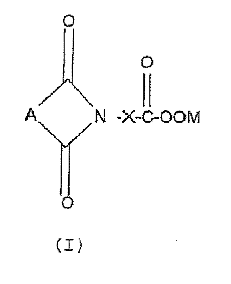 aufweisen, worin A für eine unter den folgenden ausgewählte Gruppe steht: 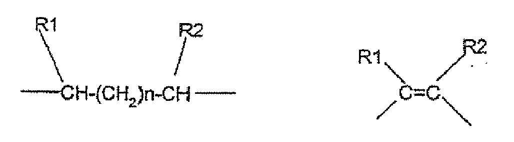 oder 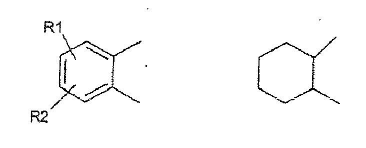 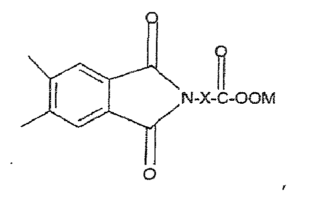 worin:
n für eine ganze Zahl mit einem Wert von 0, 1 oder 2 steht;
R1 eine der folgenden Bedeutungen besitzt: Wasserstoff, Chlor, Brom, C₁-C₂₀-Alkyl, C₂-C₂₀-Alkenyl, Aryl oder Alkylaryl,
R2 für Wasserstoff, Chlor, Brom oder eine unter den folgenden ausgewählte Gruppe steht: -SO₃M, -CO₂M, -CO₃M oder -OSO₃M,
M Wasserstoff, ein Alkalimetall, Ammonium oder ein Äquivalent eines Erdalkalimetalls bedeutet,
X für C₁-C₁₉-Alkylen oder Arylen steht.

3. Verfahren nach Anspruch 2, bei dem es sich bei der Imidoalkanpercarbonsäure umε-Phthalimidoperoxyhexansäure handelt.

4. Verfahren nach den Ansprüchen 1 bis 3, bei dem die in den verdünnten Zusammensetzungen enthaltenen Imidoalkanpercarbonsäuren eine Auflösungszeit t₉₉ im Waschbad bei einer Temperatur von 20°C von weniger als fünf Minuten aufweisen.

5. Verfahren nach den Ansprüchen 1 bis 4, bei dem man in Schritt I) nichtionische und/oder anionische Tenside derart zugibt, daß die Gesamtkonzentration an Tensiden, ausgedrückt als Gewichtsprozentanteil, mindestens 0,001% beträgt.

6. Verfahren nach den Ansprüchen 1 bis 5, bei dem man in Schritt II) eine Scherung zwischen 50.000 und 100.000 s⁻¹ verwendet.

7. Verfahren nach den Ansprüchen 1 bis 5, bei dem man in Schritt II) eine Scherung zwischen 5000 und 30.000 s⁻¹ in Gegenwart einer Gesamtmenge an nichtionischen und/oder anionischen Tensiden, ausgedrückt als Gewichtsprozentanteil, von mindestens 0,01% und vorzugsweise mindestens 0,03% verwendet.

8. Verfahren nach den Ansprüchen 1 bis 7, bei dem man in Schritt III) das Verdünnen mit einer wäßrigen Lösung eines Suspendiermittels durchführt, so daß man eine Endkonzentration in der verdünnten Zusammensetzung von 0,01% bis 0,6% und vorzugsweise von 0,05% bis 0,3% Suspendiermittel erhält.

9. Verfahren nach Anspruch 8, bei dem es sich bei dem Suspendiermittel um Xanthan handelt.

10. Verfahren nach den Ansprüchen 8 und 9, bei dem man Schritt III) bei einer Temperatur zwischen 5°C und 35°C durchführt.

11. Nach dem Verfahren gemäß einem der Ansprüchr 1 bis 10 erhältliche verdünnte wäßrige Lösungen, die, ausgedrückt als Gewichtsprozentanteil, eine Menge von < 7 % Imidoalkanpercarbonsäuren enthalten.

12. Verwendung der verdünnten wäßrigen Lösungen gemäß Anspruch 11 bei Wasch- und Reinigungsanwendungen.

13. Verwendung der verdünnten wäßrigen Lösungen gemäß Anspruch 11 bei Desinfektionsanwendungen.

14. Verwendung der verdünnten wäßrigen Lösungen nach den Ansprüchen 12 oder 13, bei der man die verdünnten wäßrigen Lösungen mit Hilfe von Dispersem (Triggern) aufbringt.

15. Verwendung der verdünnten wäßrigen Lösungen nach den Ansprüchen 12 bis 14 bei Wasch- und Reinigungsmittelanwendungen bei einer Temperatur von etwa 10°C bis 30°C.

## Revendications

1. Procédé pour obtenir des compositions aqueuses diluées contenant une quantité < 7% en poids d'acides imidoalcanepercarboxyliques, commençant par des compositions aqueuses concentrées contenant une quantité ≥ 7% desdits peracides sous forme β-cristalline, lesdites compositions concentrées étant obtenues à partir des acides imidoalcanepercarboxyliques sous forme α-cristalline, comprenant les étapes suivantes :
I) dilution de la composition aqueuse concentrée d'acides imidoalcanepercarboxyliques (C) avec une solution aqueuse (D) ayant un pH compris entre 2 et 5, de préférence de 3 à 4, et de manière particulièrement préférée de 3,2 à 3,7, dans une proportion (C):(D) exprimée en parties en poids comprise entre 0,1:10 et 10:0,2, en opérant à des températures comprises entre 4°C et 50°C et de préférence entre 10°C et 25°C;
II) application à la composition aqueuse diluée obtenue dans I) d'une force de cisaillement d'au moins 5 000 s⁻¹, jusqu'à obtention d'une viscosité dynamique constante;
III) dilution de la composition aqueuse diluée obtenue dans II) afin d'obtenir une concentration en acide imidoalcanepercarboxylique < 7% en poids;
IV) en option, homogénéisation finale de la composition.

2. Procédé selon la revendication 1, dans lequel les acides imidoalcanepercarboxyliques ont pour formule (I) 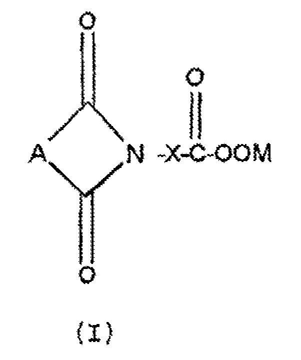 dans laquelle A indique un groupe choisi parmi les suivants : 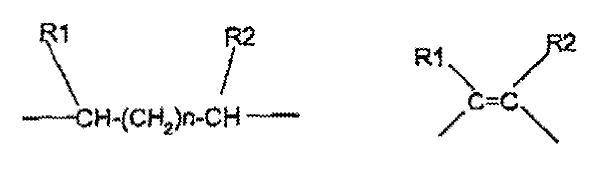 ou 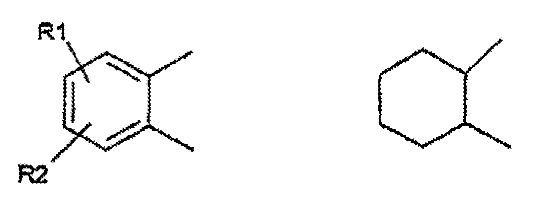 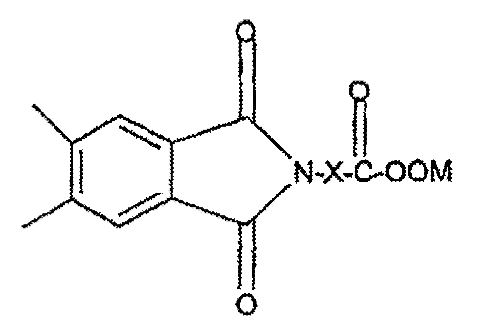 dans lesquels :
n est un nombre entier valant 0, 1, ou 2,
R1 prend l'une des significations suivantes : un atome d'hydrogène, de chlore, de brome, un groupe alkyle en C₁ à C₂₀, alcényle en C₂ à C₂₀, aryle ou alkylaryle,
R2 représente un atome d'hydrogène, de chlore, de brome ou un groupe choisi parmi les suivants : -SO₃M, -CO₂M, -CO₃M ou -OSO₃M,
M signifie un atome d'hydrogène, un métal alcalin, un ion ammonium ou un équivalent d'un métal alcalino-terreux,
X indique un groupe alkylène en C₁ à C₁₉ ou un groupe arylène.

3. Procédé selon la revendication 2, dans lequel l'acide imidoalcanepercarboxylique est l'acide ε-phtalimidoperoxyhexanoïque.

4. Procédé selon les revendications 1 à 3, dans lequel les acides imidoalcanepercarboxyliques contenus dans les compositions diluées ont une durée de dissolution t₉₉ dans le bain de lavage à une température de 20°C, inférieure à 5 minutes.

5. Procédé selon les revendications 1 à 4, dans lequel, à l'étape I), des tensio-actifs non ioniques et/ou anioniques sont ajoutés de sorte que la concentration totale en tensio-actifs, exprimée sous forme d'un pourcentage en poids, ne soit pas inférieure à 0,001%.

6. Procédé selon les revendications 1 à 5, dans lequel, à l'étape II), un cisaillement compris entre 50 000 et 100 000 s⁻¹ est employé.

7. Procédé selon les revendications 1 à 5, dans lequel, à l'étape II), un cisaillement de 5 000 à 30 000 s⁻¹ est employé en présence d'une quantité totale de tensio-actifs non ioniques et/ou anioniques, exprimée sous forme de pourcentage en poids, qui n'est pas inférieure à 0,01% et de préférence pas inférieure à 0,03%.

8. Procédé selon les revendications 1 à 7, dans lequel, à l'étape III), la dilution est réalisée avec une solution aqueuse d'un agent de mise en suspension de sorte à avoir une concentration finale dans la composition diluée de 0,01% à 0,6%, et de préférence de 0,05% à 0,3% d'agent de mise en suspension.

9. Procédé selon la revendication 8, dans lequel l'agent de mise en suspension est la gomme xanthane.

10. Procédé selon les revendications 8 et 9, dans lequel l'étape III) est réalisée à une température comprise entre 5°C et 35°C.

11. Composition aqueuse diluée contenant une quantité < 7%, exprimée sous forme de pourcentage en poids, d'acides imidoalcanepercarboxyliques, qui peuvent être obtenus à l'aide du procédé selon l'une quelconque des revendications 1 à 10.

12. Utilisation des compositions aqueuses diluées selon la revendication 11 dans des applications de détergence.

13. Utilisation des compositions aqueuses diluées selon la revendication 11 dans des applications de désinfection.

14. Utilisation des compositions aqueuses diluées selon les revendications 12 ou 13, dans laquelle les compositions aqueuses diluées sont appliquées au moyen de disperseurs (gâchettes).

15. Utilisation des compositions aqueuses diluées selon les revendications 12 à 14, employées dans des applications de détergence à une température d'environ 10°C à 30°C.
